# EUROPEAN PATENT SPECIFICATION

(11) **EP 1 135 090 B1**
(45) Date of publication and mention of the grant of the patent: **04.08.2004**
(21) Application number: 99946210.4
(22) Date of filing: 22.09.1999
(51) Int. Cl.: A61F 13/20

(54) **TAMPON WITH INDICATION MEANS**
TAMPON MIT INDIKATOREN
TAMPON AVEC MOYEN INDICATEUR

(30) Priority: 28.09.1998 FI 982079
(43) Date of publication of application: 26.09.2001
(73) Proprietor: Tojkander, Erja, Anita, Helena, 18200 Heinola (FI)
(72) Inventor: Tojkander, Erja, Anita, Helena, 18200 Heinola (FI)
(74) Representative: Laitinen, Pauli Sakari
(86) International application number: PCT/FI1999/000779
(87) International publication number: WO 2000/018345

(56) References cited:
- US-A- 2 112 021
- US-A- 3 794 024
- US-A- 5 217 444
- US-A- 5 273 521
- US-A- 5 468 236
- US-A- 5 769 813

## Description

The present invention relates to a tampon for use inside the body, which tampon has a separate indicator area added to the moisture protection/leak protection layer, to make it safer to use. The tampon's indicator marking shows changes in body moisture or acidity balance, or the presence of toxins, without the tampon having to be removed or treated with a colouring substances after removal.

Tampons used in tamponing are generally made from highly absorbent material, such as cotton or other highly absorbent fibres. In medicine, tampons are mainly used to absorb various discharges of body fluids and haemorrhaging after operations and curettage. However, they are mostly used to absorb normal menstrual flow, i.e. as sanitary towels.

Used as a sanitary towel, a tampon is inconspicuous and easy to use, indeed it is so inconspicuous that the interval between changes can become unnecessarily long, especially if the tampon is absorbent enough to permit infrequent changes. Under unfavourable circumstances, such long intervals may create a high risk of an explosive growth of anaerobic bacteria, making the tampon a dangerous time-bomb. However, changes in the pH of the vagina are reckoned to be relatively slow, provided conditions do not deteriorate unnecessarily. If changes in pH begin, a reliable method must be used to indicate them, making it possible to know if it is safe to use a tampon, for instance. The use of such an indicator method will make tampons safer than before.

Changes in the vaginal pH can cause many different diseases, such as Tricomonas vaginitis, which is caused by the flagellata Tricomonas vaginalis. This disease raises the normal vaginal pH, which is < 4,7, to at least 5,5. Non-specific colpitis too raises the vaginal pH to> 5,5. No definite cause of this disease has been found, but gardnerella vaginalis is one possible suspect. So-called colpitis senilis also causes an increase in pH.

The most usual syndrome in tampon users, which is actually due to tampon use as such, is the toxic shock syndrome (TSS), usually caused by toxins of staphylococcus aureus. There are very few ways to predict these serious and irritating diseases, without using difficult laboratory tests. Generally, however, it can be assumed that these diseases develop relatively slowly and that using a tampon in connection with them helps the disease to develop to a more dangerous stage. All these changes in pH, as well as the presence of toxins, are indicated by a change in the vaginal pH to greater alkalinity. Thus, indicator colours differing from the colour of normal body fluids, and which react to different toxins and are applicable to several pH ranges, can be used to show changes. For example, methyl orange has a colour variation range with pHs from 3.1 to 4.1, with the acid end coloured red and the alkali end orange. The pHs of the colour variation of methyl red range from 4.4 to 6.2, with red at the acid end and yellow at the alkali end. Litmus's range of variation has pHs of 5.0 to 8.0, with red at the acid end and blue at the alkali. For example, the use of these indicator substances has the practical advantage that a sanitary towel is naturally coloured red by menstrual blood. Thus, its natural colour indicates a normal vaginal pH, with the tampon's colouring substance only becoming visible in situations, in which a tampon should not be used. In addition, users will notice an unnatural marking colour more easily. This same principle of using colouring substances can be applied to indicate toxins.

Previous attempts have been made to solve the problem referred to above. One of them being a tampon construction intended to indicate the presence of microbes, which is disclosed in USA patent 3864213. This tampon contains a culture medium to determine the presence of microorganisms, but must first be removed and treated with a colouring substance, before the determination can be carried out.

The basic idea of the present invention avoids this problem, creating an indicator tampon, which can even be interpreted in use, to determine changes in the vaginal pH or the presence of increased toxins. The invention is intended to make tampons safer, telling the tampon user to seek professional care in time, if any deviation from the conditions in which use of a tampon is safe is observed. The characteristic features of the tampon are disclosed in the accompanying Claims. One such feature is the addition of an indicator area to the moisture/leak protection layer on the surface of the tampon, so that moisture will reach the indicator area and bring with it microbes or various toxins that cause changes in the pH. As the tampon's indicator areas are protected under its moisture/leak protection layer, it is much less likely that external microbes and toxins will reach the marking area, when the tampon is being handled, e.g., when being set in place. The moisture/leak protection layer ensures that the indicator area is only exposed to microbes or toxins that are transported by a great deal of moisture, such as blood or other body fluids, thus ensuring the indication of real conditions, and not of, for example, only dirty hands. When the tampon is being pressed into shape, special suction channels can be formed in it to lead moisture, microbes and possible toxins as quickly as possible to the tampon's indicator area. In this way, any change in the indicator area due to them will be seen as soon as possible.

In the following, the invention is described in greater detail by referring to the accompanying drawings, in which;
Figure 1 shows a conventional tampon pressed into shape, with a moisture/leak protection layer at its lower end, and guide and suction channels inside it;
Figure 2 shows a tampon, in which the indicator markings are visible;
Figure 3 shows differently shaped markings;
Figure 4 shows a cross-section of the tampon through the moisture/leak protection layer;
Figure 5 shows the tampon placed ready in the vagina;
Figure 6 shows a cross-section of the lower end of the tampon; and
Figure 7 shows a tampon, with indicator marking areas shaped in its lower end.

Though the invention is described in the following only in connection with a tampon intended to collect menstrual flow, it should be understood that the same principles can also apply to other uses. It should also be understood that the disclosure of the invention with the aid of embodiments in no way limits the invention to these embodiments.

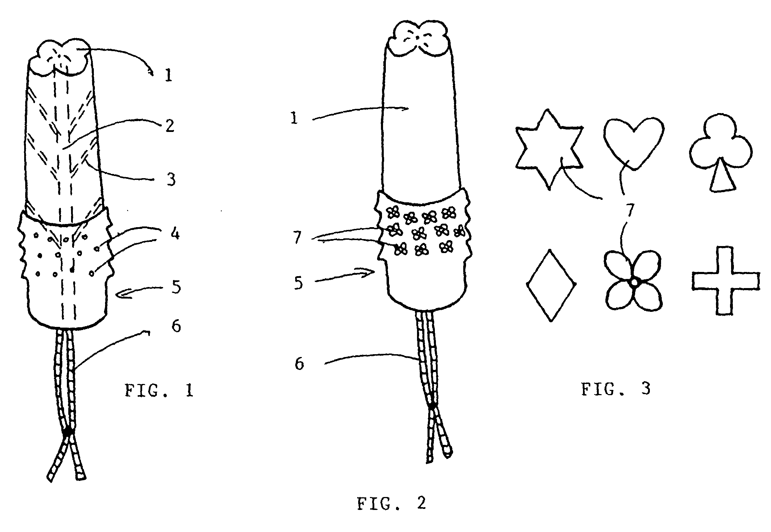

Figure 1 shows a normal tampon pressed into shape, which can be placed in the vagina either with an applicator or a finger. Part 1 of the figure comprises absorbent material forming the core of the tampon, its absorbent layer, which continues to the lower end of the tampon beneath moisture/leak protection layer 5.

When the tampon is being pressed into shape, various hollow, tube-like shapes can also be pressed it is, to assist the absorption of liquid. These can be like guide channel 2, which runs from end to end of the tampon, and small channels in the side of the tampon like suction channel 3, which is capillary and improves the transportation of moisture, microbes and toxins to the indicator marking area 7 under moisture/leak protection layer 5.

Small suction holes 4 in the actual moisture/leak protection layer 5 lead moisture, microbes and toxins directly from the vagina to indicator marking area 7. Information on the vaginal pH level or on toxins that are present can also be led directly outside the vagina along removal string 6, if this is treated with an indicator substance.

Figure 2 shows tampon 1, which has been pressed into shape, with various indicator markings 7, created by indicator colours, visible in moisture/leak protection layer 5.

Figure 3 shows many different possible markings, which, if desired, can be used to display a particular indicator marking 7 on layer 5 on the surface of the tampon, to indicate a change in the acidity of the vagina or the presence of toxins.

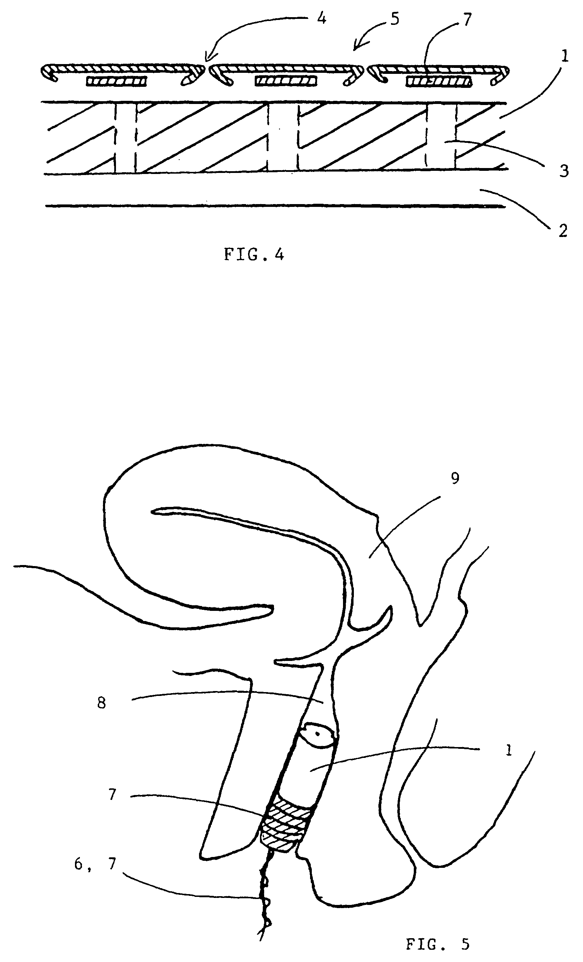

Figure 4 shows a cross-section of tampon 1 through its moisture/leak protection layer 5. Special small holes 4, made in moisture/leak protection layer 5 can be seen in the figure. At holes 4, the moisture/leak protection material is pushed inside the tampon with a sharp instrument, so that the edges formed by the holes are turned inwards. This allows moisture, microbes and toxins coming from the vagina side to easily pass under layer 5, but prevents moisture coming from the core of the tampon itself from seeping outwards, as the moisture absorbed by the tampon expands it from inside the absorbent layer and pushes the already wet core against the inwardly turned edges of the holes, thus blocking the passage of moisture from the core to the outside of the tampon. Just as moisture can be led to indicator marking area 7 through the tampon's holes 4, it can also be led to guide channel 2 in the core of tampon 1, and to indicator marking area 7, by means of special smaller channels 3, causing the desired reaction in the marking substance.

Figure 5 shows tampon 1 in place in vagina 8, with tampon removal string 6 connected to the indicator making substance or area 7. Moisture, microbes or toxins are led as quickly as possible to indicator area 7 through the guide channel in the tampon, thus allowing microbes or toxins in the uterus 9 above to also be easily discerned from the change in the colour of the indicator substance, either in layer 5 on top of tampon 1 itself, or even in removal string 6 outside the body.

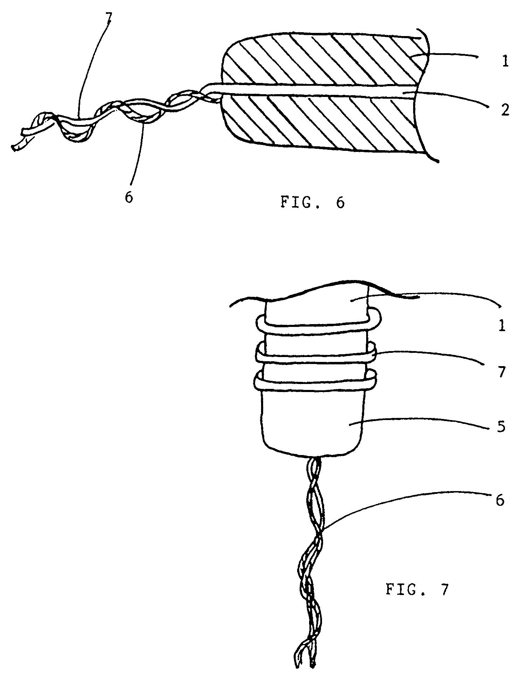

Figure 6 shows a cross-section of the removal string end of the tampon, in which 1 is the actual absorbent material of the tampon, with guide channel 2 inside it. A special indicator marking 7, which is led from channel 2 directly to removal string 6 extending outside the body, allows changes in the marking substance to be observed outside the body.

Figure 7 shows a projection of the removal string end of the tampon, with special shapes improving retention formed in the moisture/leak protection layer 5 in the lower end of tampon 1. These are also treated with an indicator substance and thus act as indicator markings 7.

The invention can be varied in many ways, without altering its basic principle. For instance, many different techniques and materials can be used to protect the area of indicator marking from external microbes or toxins. Such materials include various impregnants and waxes, which can be use to protect the outer layer and make the indicator marking substance area react only with moisture and microbes coming from the core of the tampon, ensuring that microbes travel with the moisture from the vagina through the core of the tampon to the area of the indicator substance.

## Claims

1. A tampon, used internally, intended to show changes in the body's internal pH caused by toxins, comprising at least one fluid-absorbing mass or layer (1), a moisture/leak protection layer (5), and a marking substance (7) to indicate changes, **characterized in that** indicator substance or material (7) lies on top of the tampon's absorbent material (1) and under a moisture/leak protection layer (5) of a moisture-isolating substance or material.

2. A tampon according to Claim 1, **characterized in that** guide channels (2, 3) are formed in the tampon's absorbent material (1).

3. A tampon according to Claim 2, **characterized in that** it includes at least one longitudinal guide channel (2) and possibly other guide channels (3) running at an angle.

4. A tampon according to Claim 3, **characterized in that** at least some of channels (2, 3) terminate in the immediate vicinity of the indicator material.

5. A tampon according to Claim 1, **characterized in that** the moisture/leak protection layer or material (5) includes holes (4).

6. A tampon according to Claim 5, **characterized in that** holes (4) have edges turned towards the interior of the tampon.

7. A tampon according to Claim 1, **characterized in that** its indicator material (7) is located in such a way that changes in its colour or other properties can be seen from or through the surface of the moisture/leak protection layer (5).

8. A tampon according to Claim 1, **characterized in that** indicator material (7) forms symbols that change colour or have other observable properties.

9. A tampon according to Claim 1, **characterized in that** moisture/leak protection material (5) includes raised shapes.

10. A tampon according to Claim 2, **characterized in that** at least one guide channel (2, 3) is intended to end very close to the lower end of the tampon and that the channel is connected to a device containing indicator material and extending outside the tampon.

11. A guide channel (2) tampon according to Claim 10 **characterized in that** the device containing indicator material is a string or tape extending outside the tampon (1).

## Patentansprüche

1. Innerlich eingesetzter Tampon, der die Änderungen des durch Toxine verursachten Körper-inneren pH's anzeigen soll, bestehend zumindest aus einer Flüssigkeit absorbierenden Masse oder Schicht (1), einer Feuchtigkeits/Leckschutzschicht (5) und einer Markiersubstanz (5) zur Anzeige von Änderungen, **dadurch gekennzeichnet, dass** die/das Indikatorsubstanz- oder -material (7) über dem Absorptionsmaterial (1) des Tampons und unter einer Feuchtigkeits/Leckschutzschicht (5) einer/eines Feuchtigkeits-isolierenden Substanz oder Materials liegt.

2. Tampon nach Anspruch 1, **dadurch gekennzeichnet, dass** im Absorptionsmaterial (1) des Tampons Führungskanäle (2, 3) ausgebildet sind.

3. Tampon nach Anspruch 2, **dadurch gekennzeichnet, dass** er zumindest einen in der Längsrichtung verlaufenden Führungskanal (2) und möglicherweise unter einem Winkel verlaufende Führungskanäle (3) umfasst.

4. Tampon nach Anspruch 3, **dadurch gekennzeichnet, dass** zumindest einige der Kanäle (2, 3) in der unmittelbaren Nähe des Indikatormaterials münden.

5. Tampon nach Anspruch 1, **dadurch gekennzeichnet, dass** die/das Feuchtigkeits/Leckschutzschicht oder -material (5) Löcher (4) umfasst.

6. Tampon nach Anspruch 5, **dadurch gekennzeichnet, dass** die Ränder der Löcher (4) zum Inneren des Tampons hin gebogen sind.

7. Tampon nach Anspruch 1, **dadurch gekennzeichnet, dass** sein Indikatormaterial (7) auf solche Weise platziert ist, dass Änderungen seiner Farbe oder anderer Eigenschaften von oder durch die Oberfläche der Feuchtigkeits-/Leckschutzschicht (5) ersichtlich sind.

8. Tampon nach Anspruch 1, **dadurch gekennzeichnet, dass** das Indikatormaterial (7) Symbole bildet, die die Farbe ändern oder andere erkenntliche Eigenschaften haben.

9. Tampon nach Anspruch 1, **dadurch gekennzeichnet, dass** das Feuchtigkeits/Leckschutzmaterial (5) erhöhte Formen umfasst.

10. Tampon nach Anspruch 2, **dadurch gekennzeichnet, dass** zumindest ein Führungskanal (2, 3) sehr nah am unteren Ende des Tampons münden soll und dass der Kanal mit einer Vorrichtung verbunden ist, die Indikatormaterial beinhaltet und sich bis zur Außenseite des Tampons erstreckt.

11. Tampon mit Führungskanälen (2) nach Anspruch 10, **dadurch gekennzeichnet, dass** die Indikatormaterial beinhaltende Vorrichtung ein Streifen oder ein Band ist, der/das sich bis zur Außenseite des Tampons (1 ) erstreckt.

## Revendications

1. Tampon pour l'utilisation interne, destiné à indiquer les modifications internes du corps, causés par le ph et des toxines, qui comprend au moins une masse ou une couche (1) absorbant du liquide, une couche de protection contre l'humidité / anti-fuites (5) et une substance marqueur (7) pour indiquer les modifications, **caractérisé en ce que** l'indicateur ou la substance marqueur (7) se trouve sur la matière absorbante (1) du tampon et sous la couche de protection contre l'humidité / anti-fuites (5) ou sous la substance ou la matière isolant l'humidité.

2. Tampon selon la revendication 1, **caractérisé en ce que** des canaux conducteurs (2, 3) ont été formés dans la matière absorbante (1) du tampon.

3. Tampon selon la revendication 2, **caractérisé en ce qu**'il contient au moins un canal conducteur longitudinal (2) et éventuellement d'autres canaux conducteurs (3) diagonaux.

4. Tampon selon la revendication 3, **caractérisé en ce qu'**au moins une partie des canaux (2, 3) se terminent à la proximité immédiate de la matière indicatrice.

5. Tampon selon la revendication 1, **caractérisé en ce que** la couche de protection contre l'humidité / anti-fuites ou la matière (5) est percée de trous (4).

6. Tampon selon la revendication 5, **caractérisé en ce que** les trous (4) ont des bords pliés vers l'intérieur du tampon.

7. Tampon selon la revendication 1, **caractérisé en ce que** sa matière indicatrice (7) est située de telle façon que les modifications de sa couleur ou d'une autre caractéristique sont visibles sur la surface de la couche de protection contre l'humidité / anti-fuites (5) ou à travers celle-ci.

8. Tampon selon la revendication 1, **caractérisé en ce que** sa matière indicatrice (7) forme des symboles modifiant leur couleur ou leurs autres caractéristiques observables.

9. Tampon selon la revendication 1, **caractérisé en ce que** la matière de protection contre l'humidité / anti-fuites (5) contient des formations en relief.

10. Tampon selon la revendication 2, **caractérisé en ce qu**'au moins un canal conducteur (2, 3) est destiné à se terminer à la proximité immédiate du bout inférieur du tampon, et qu'un moyen contenant de la matière indicatrice et s'étendant à l'extérieur du tampon, a été attaché à ce canal.

11. Canal conducteur (2) selon la revendication 10, **caractérisé en ce que** le moyen contenant de la matière indicatrice est une ficelle ou un ruban s'étendant à l'extérieur du tampon (1).
